# EUROPEAN PATENT APPLICATION

(11) **EP 1 415 664 A1**
(43) Date of publication of application: **06.05.2004**
(21) Application number: 02024200.4
(22) Date of filing: 30.10.2002
(51) Int. Cl.: A61K 38/55, A61K 48/00, A61P 17/02

(54) **Use of alpha 1-antichymotrypsin in combination with alpha-1-antitrypsin for treating/preventing diabetes associated or poorly healing arterial wounds**

(71) Applicant: Switch Biotech Aktiengesellschaft, 82152 Martinsried (DE)
(72) Inventor: Halle, Jörn-Peter, 82377 Penzberg (DE); Goppelt, Andreas, 80636 München (DE)
(74) Representative: Krauss, Jan

(57) **Abstract**

The invention relates to the use of alpha 1-antichymotrypsin (ACT) polypeptides , functional variants thereof and/or nucleic acids encoding them, or of a cell which is expressing an ACT polypeptide or a nucleic acid encoding it in combination with alpha-1-antitrypsin (AAT) polypeptides, functional variants thereof or nucleic acids encoding them, or of a cell which is expressing an AAT polypeptide, or a nucleic acid encoding it, for the treatment and/or prevention of poorly healing diabetes-associated and/or poorly healing arterial wounds.

## Description

The invention relates to a use of alpha 1-antichymotrypsin (ACT) polypeptides , functional variants thereof and/or nucleic acids encoding them, or of a cell which is expressing an ACT polypeptide or a nucleic acid encoding it in combination with alpha-1-antitrypsin (AAT) polypeptides, functional variants thereof or nucleic acids encoding them, or of a cell which is expressing an AAT polypeptide, or a nucleic acid encoding it.

Skin wounds in healthy patients normally heal without any complications. However, a large number of temporal and spatial changes in the cell composition of the skin is required in order to achieve complete healing of the tissue. This process can last up to 2 years and is always associated with scar formation in non-fetal tissue. This points to the enormous complexity of the wound healing process in the skin. During the wound healing process, it is possible to distinguish different temporal and partially overlapping phases: coagulation, inflammation, proliferation and remodeling (The Physiology of Wound Healing, 1998, Oxford Institute for Continuing Education). During coagulation, blood platelets aggregate and release growth and coagulation factors. A fibrin matrix is formed, thus enabling cells to migrate into the wound. Approximately 5-7 days after injury, an inflammatory reaction is triggered by the migration of a variety of cell types into the wound, in particular neutrophilic granulocytes and monocytes which release the mediators of the inflammatory reaction. During the proliferation phase blood vessels are restored, damaged tissue is regenerated, and the regenerated tissue is remodeled. The processes during the proliferation phase comprise, in particular, neovascularization, fibroblast proliferation and reepithelialization due to the proliferation and differentiation of keratinocytes. The fibroblasts secrete several growth factors, such as PDGF and TGF-beta, which in turn regulate the synthesis and deposition of components of the extracellular matrix (ECM), such as fibronectin, laminin, glycosaminoglycans and collagen.

During the reorganization of the tissue, the ECM components, particularly collagen, are rearranged. As a result of collagen being continuously degraded and newly synthesized, the reepithelialized wound can mature, and a flat scar is formed within 2 years. Again, a large number of growth factors and chemoattractants are required for reconstructing the tissue in a coordinated manner. Thus, interleukin 1, TNF-beta and interferon-gamma influence the secretion of the ECM components. TGF-beta, PDGF and FGF are also essential for remodeling.

However, in addition to the processes which contribute to the reconstruction of destroyed structures, proteolytic processes are also important for wound healing. Proteolytic processes participate in the removal of cell debris and the degradation of intermediate structures, such as fibrin matrix. Thus, a large number of proteases are active at the edge of the wound (Martin et al., 1997, Science, 276: 75-81). For example, plasminogen is activated by plasminogen activator and the urokinase-type plasminogen activator is upregulated in migrating keratinocytes, enabling them to degrade the fibrin matrix located ahead of them. This is consistent with the observation that plasminogen-knock-out mice exhibit virtually no reepithelialization. Metalloproteinases (MMP) also play an important role. MMP9 (gelatinase B), MMP (interstitial collagenase) and MMP 10 (stromelysin-2) are activated at various time points and are characterized by different substrate specificities. Neutrophilic granulocytes and monocytes (macrophages) also secrete proteases (Dome et al., 1999, Wound Rep. Reg. 7: 433-441; Shapiro et al., J. Rheumatol. Suppl., 1991, 27; 95-98). Monocytes contain the intracellular serine proteases elastase and cathepsin G and secrete small amounts of metalloproteinases. On the other hand, in differentiating mononuclear phagocytes, the expression of cathepsin G is suppressed and the expression of collagenase is delayed. It has been observed that, in mature macrophages, the expression of metalloproteinases is powerfully induced following stimulation (Shapiro et al., J. Rheumatol. Suppl., 1991, 27: 95-98).

With regard to chronic wounds, it is the matrix metalloproteinases which have become the focus of particular interest. Thus, the amounts of collagenolytic activity which are found in the exudates of chronic wounds are significantly increased compared to those found in the exudates of surgical wounds or open skin wounds (Yager et al., 1996, J. Invest. Dermatol. 107: 743-748). For example, a variety of investigations provide evidence that the amount of MMP-1 is markedly increased in chronic wounds and that MMP-1 is the predominant collagenase in the exudates of chronic wounds (Vaalamo et al., 1997, J. Invest. Dermatol., 109; 96-101; Nwomeh et al., 1999. J. Surg. Res., 81; 189-195). MMP8 is also expressed more strongly in chronic wounds (Yager et al., 1996, J. Invest. Dermatol. 107: 743-748; Nwomeh et al., 1999, J. Surg. Res., 81: 189-195). In addition to the MMPs belonging to the interstitial collagenase class, other MMPs, namely the gelatinases MMP2 and MMP9 and the stromelysins MMP3, MMP 10 and MMP11, have been demonstrated to be present in increased amounts in chronic wounds (Nagase and Woessner, 1999, J. Biol. Chem., 274; 21491-21494). It has been postulated that serine protease activity, namely elastase activity, is present in chronic wounds, with this activity degrading fibrin and fibronectin (Palolahti et al., 1993, Exp. Dermatol., 2: 29-37; Rao et al., 1995, J. Invest. Dermatol., 105; 572-578; Grinnell and Zhu, 1996, J. Invest. Dermatol., 106: 335-341; Herrick et al., 1997, Lab. Invest. 77: 281-288).

Another serine protease, cathepsin G, has been detected in the granulation tissue in chronic decubitus ulcers (Rogers et al., 1995; Wound Rep. Reg., 3: 273-283). In contrast, it was not possible to observe any increased amounts of cathepsin G in venous foot ulcers (Weckroth et al., 1996, J. Invest. Dermatol., 106: 1119-1124). These apparently contradicting results indicate that the term "chronic skin wounds" encompasses completely different diseases with differing pathogenetic backgrounds. In general, diabetic ulcers, venous ulcers, arterial ulcers and decubitus ulcers are discriminated. Decubitus ulcers are very deep wounds which are accompanied by necrosis, infection and maceration of the tissue. They are formed due to prolonged pressure to a given skin area. By contrast, venous ulcers are rather superficial and are caused by venous stasis whereas arterial ulcers are frequently caused by arterial occlusion diseases. Diabetic ulcers, in turn, are ulcers which often arise in diabetic patients. Among a large number of diabetes-associated complications, the late complications of diabetes also comprise characteristic changes in the skin such as frequent infections, trophic disturbances and *necrobiosis lipoidica.* These changes can develop into poorly healing ulcers, frequently as the result of microangiopathic disturbances. The epidemiological importance of these diseases is clear when the following survey findings are considered: 25% of patients suffering from type II diabetes frequently develop chronic ulcers ("diabetic foot") with about half of them requiring elaborate in-patient treatment. Nevertheless these ulcers heal poorly in the end. Diabetic foot causes more hospitalization than does any other complication associated with diabetes. The number of these cases associated with diabetes type I and II is on the increase and represents approx. 2.5% of all hospital admissions.

It has been postulated that a disturbed, excessively powerful proteolytic activity is responsible for the poor healing in chronic wounds (Yager et al., 1999, Wound Rep. Reg., 7: 433-441). In normally healing wounds, an equilibrium between proteolytic and antiproteolytic activity is brought about by a broad range of protease inhibitors. This theory is supported by the observation of reduced amounts of protease inhibitors within chronic wounds such as the metalloproteinase inhibitor TEMP-1, the nonspecific protease inhibitor alpha2-macroglobulin and the elastase inhibitor alphal-protease inhibitor (Yager et al., 1997, Wound Rep. Reg., 5:23-32; Grinnell et al., J. Invest. Dermatol., 110; 771-776; Bullen et al., J. Invest. Dermatol., 104; 236-240; Rao et al., 1995; J. Invest. Dermatol., 105: 572-578; Grinnell and Zhu, 1996, J. Invest. Dermatol., 106: 335-341). It is thought that this deficiency could lead to an incomplete "antiprotease shield" with respect to those protease inhibitors. As a consequence, the turnover rate of collagen is faster than its synthesis. These results are confirmed by other studies (Nwomeh et al., 1999, J. Invest. Dermatol., 81: 189-195; Vaalamo et al., 1996, Br. J. Dermatol., 135; 52-59; Witte et al., 1998, Surgery 124: 464-470). It has therefore been suggested to increase the antiprotease shield by raising or inducing the expression of the protease inhibitors which are reduced or lacking , respectively, by administering exogenous protease inhibitors. A large number of inhibitors of the metalloproteinases have been developed on the basis of this hypothesis (e.g. WO200073295; US6166084; WO200105397; WO200063165; WO200046189; WO200044723; DE19851184; US6071903; EP-1004578; EP-949246; WO9858925; EP-878467); none of which has, however, been authorized as a pharmaceutical which is intended for treatment chronic skin wounds and which intervenes in the protease-antiprotease equilibrium. Furthermore, research to date did not discriminate between the various diseases which are grouped under the term "chronic ulcers". For this reason, the results of the studies cannot readily be transferred to other disturbances within the wound healing process. Thus up until now, there have been no effective therapies for chronic wound healing disturbances. Established forms of therapy are restricted to physical support of the wound healing (e.g. dressings, compresses and gels), to the scraping out of necrotic tissue and to the transplantation of skin tissues, cultured skin cells and/or matrix proteins. In recent years, growth factors have been tested for their ability to improve wound healing without, however, being able to improve the conventional therapy in a decisive manner.

In view of the strongly increasing number of diabetes II patients world-wide, and the large number of patients who are suffering from arterial ulcers, there is a large demand for novel active compounds which crucially improve the healing of poorly healing diabetes-associated and poorly healing arterial wounds. Therefore, the object of the present invention was to find a novel active and stable therapeutical composition which crucially improves the healing of poorly healing diabetes-associated and poorly healing arterial wounds.

It was found that the activity of the ACT polypeptides is selectively decreased in poorly-healing diabetic wounds compared to the observed increase in activity of ACT in normally healing wounds as well as in venous ulcers. Also, ACT was found to be specifically suitable for treating diabetic organism compared to non-diabetic organisms. However, the environment of the wound is extremely aggressive for polypeptides; i.e. ACT can be inactivated especially by elastase which is present in wounds in high amounts (see Example 2). In order to protect ACT against proteolytic inactivation and/or degradation by elastase, an ACT polypeptid and/or nucleic acids encoding this or cells expressing an ACT polypeptide or nucleic acids encoding this in combination with an AAT polypeptide and/or nucleic acids encoding this or cells expressing an AAT polypeptide or nucleic acids encoding this can be used for the treatment and/or prevention of diabetes-associated and/or arterial poorly healing wounds in mammals. In one preferred embodiment, the combination is achieved by a single ACT/AAT hybrid polypeptide, wherein the polypeptide has both elastase and chymotrypsin inhibitory function; especially preferred is a Lex032 polypeptide according to SEQ ID No. 9. Such hybrid polypeptides are e.g. known from WO95/27055 and comprise ACT polypeptides wherein the amino acid at position 358 of the mature protein is mutated to methionine, isoleucine, valine, alanine, aspartic acid, threonine and glutamic acid. Also disclosed are such polypeptides wherein the amino acids 356 through 361 of mature human ACT are substituted with Ile-Pro-XXX-Ser-Ile-Pro, wherein XXX is selected from the group of methionine, tryptophan, alanine, asparagine, aspartic acid, cysteine, glutamine, glutamic acid, glycine, histidine, isoleucine, lysine, phenylalanine, proline, serine, threonine, tyrosine and valine. Especially the polypeptide Lex032 wherein the amino acids 356 through 361 of mature human ACT are substituted with Ile-Pro-Met-Ser-Ile-Pro was found to be effective in inhibiting both elastase and chymotrypsin.
Experiment 1 which was carried out within the context of this invention demonstrates the specific efficacy of ACT for treatment of poorly healing wounds of diabetic mammals and poorly healing arterial wounds compared to treatment of healthy animals. Experiment 2 indicates that, although ACT polypeptides are abundant in chronic wound fluids of diabetic patients, the proportion of active ACT is extremely low, compared to wound fluids from acute wounds or chronic venous wounds. These findings surprisingly support the notion, that ACT in combination with AAT is especially suitable for treating and preventing diabetes-associated and/or arterial badly healing wounds. In a preferred embodiment, the diabetes-associated wounds are chronic diabetic wounds. In an especially preferred embodiment, the diabetes-associated wound is a diabetic ulcer. In a particularly preferred embodiment, the wounds are a diabetic ulcer or an arterial ulcer, most preferably a diabetic ulcer.
ACT is the major known endogenous protease inhibitor of cathepsin G. In humans, one ACT gene is known, with polymorphism having been described, particularly in the signal peptide sequence (Rubin, 1989, database entry). The sequence of the human ACT polypeptide sequence with signal peptide is shown in SEQ ID No. 6. The sequence of the mature human ACT polypeptide sequence without signal peptide is shown in SEQ ID No. 7. The sequence of the human ACT nucleic acid coding sequence is shown in SEQ ID No. 8. In the case of rodents, there is evidence for a large number of genes which are homologous to ACT, with the mouse serine protease inhibitor 2-2 (spi 2-2) (SEQ ID No. 1) being the functional homologue to the human ACT gene since extensive concordance exists with regard to the reactive center, the tissue distribution and the inducibility following inflammation (Inglis et al., 1991, Gene 106: 213-220). The coding sequence of spi2-2 is shown in SEQ ID No.: 2. Other members of the rodent serine protease inhibitor 2-2 family are spi2-2 and spi3 in the rat and an spi2-2 homologue from Adodemus sylvaticus (Inglis et al., see above).
Alpha-1-antitrypsin (AAT) is the major natural inhibitor of neutrophil elastase and thereby protects ACT from proteolytic degradation and inactivation by elastase. Various alleles of AAT exist, with the most frequently occurring allele shown in SEQ ID No.: 3. The sequence of the mature polypeptide without signal peptide is shown in SEQ ID No.: 4. The coding sequence for the AAT polypeptide is shown in SEQ ID No.: 5. The major physiological function of AAT is the protection of the lower respiratory tract against destruction by human leukocyte elastase. A hereditary deficiency of AAT is associated with a 20-30 fold increased risk of developing chronic obstructive disease. In skin wounds, its function as inhibitor of human neutrophil elastase is prominent. The role of AAT in wound healing has been described in the literature (e.g. Rao et al., 1995, J. Invest. Dermatol., 105: 572-578); however the specific suitability of ACT in combination with AAT for treating diabetes-associated and/or arterial wounds has not been suggested yet.
ACT was also shown to inhibit chymases in mast cells, for which reason ACT has been described in the context with allergic reactions (Lindmark and Wallengren, Allergy, 1992 47:456-458). In addition, the role of the chymase and its inhibitor ACT has been investigated in psoriatic lesions. However, the results point to the chymase only playing a subordinate role in the pathomechanism of psoriasis (Harvima et al., 1999, Acta Derm. Veneorol., 79: 98-104) and the level of chymotrypsin inhibitor activity in the uninjured skin of psoriasis patients is unaltered (Glinski et al., 1991, Arch. Dermatol. Res., 283: 224-229). According to EP0432117, ACT could be used for treatment "skin inflammations", "burns", and "dermatological conditions". However, EP0432117 does not explain the meaning, of "dermatological condition". "Skin inflammation" refers to inflammatory diseases of a skin such as psoriasis or dermatitis, which is different from mechanically damaged skin, i.e. wounds, or from deterioration in the process of the restoration of the missing tissue in the course of wound healing. "Skin inflammation" as used in EP0432117 therefore represents a pathological condition of the skin that does not encompass poorly healing arterial wounds or poorly healing diabetes-associated wounds according to the invention. By the same token the term "dermatological condition" as used in EP0432117 does not include wounds according to the invention: the term "dermatological condition" refers to conditions of the disordered skin such as blisters, cysts, macules which represent symptoms of a disorder of the skin resulting from essentially internal processes. Wounds on the other hand are caused by mechanical forces coming from outside the body.

Lex032 (SEQ ID No.: 9) acts, as a combination of ACT and AAT in a single polypeptide chain, on both cathepsin G and elastase and is used in the treatment of pancreatitis (von Dobschuetz et al., 1999, J. Pharmacol. Exp. Ther., 290: 782-8). Lex032, like the other bifunctional mutants possessing elastase-inhibiting and cathepsin G-inhibiting properties described above have been disclosed for treatment of inflammatory diseases, for example chronic wounds and psoriasis (WO 95/27055). However, the specific suitability for treating and/or preventing diabetes-associated wounds has not been described or anticipated in the prior art until now.

Taken together, although the enzymes ACT and AAT have been known for a long time in biotechnology, and have been investigated extensively from the medical point of view, the specific suitability of ACT in combination with AAT for treating and/or preventing diabetes-associated and arterial wounds has not been described yet. Also, the combination of ACT with AAT wherein a single polypeptide is used which possesses both elastase and chymotrypsin inhibitory function (e.g. Lex032) has not been described yet for for treating and/or preventing diabetes-associated and arterial wounds. This invention shows for the first time that among the large number of different possible wounds two poorly healing wounds, namely diabetes-associated and arterial ulcers, have unexpectedly the best chances for a successful therapy by ACT in combination with AAT which would not have been considered by a skilled person.

The object of the present invention is solved by the use of a alpha 1-antichymotrypsin (ACT) polypeptide, a functional variant thereof and/or a nucleic acid encoding it, or of a cell which is expressing an ACT polypeptide or a nucleic acid encoding it, in combination with an alpha-1-antitrypsin (AAT) polypeptide, functional variant thereof or nucleic acid encoding it, or of a cell which is expressing an AAT polypeptide, or a nucleic acid encoding it, for the treatment and/or prevention of poorly healing diabetes-associated and/or poorly healing arterial wounds.

Im particular, the invention relates to the use of an ACT polypeptide having a sequence selected from the group comprising SEQ ID No. 1, SEQ ID No. 6 and SEQ ID No.: 7, or a functional variant thereof or of a nucleic acid encoding it, in combination with an AAT polypeptide having a sequence selected from the group comprising SEQ ID No. 3 and SEQ ID No. 4, or a functional variant thereof or of a nucleic acid encoding it, for treatment and/or prevention of diseases which are selected from poorly healing diabetes-associated wounds and/or poorly healing arterial wounds.

In a preferred embodiment, the invention relates to the use of ACT polypeptide according to SEQ ID No.: 7, in combination with an AAT polypeptide, according to SEQ ID No.: 4 for treatment and/or prevention of diseases which are selected from poorly healing diabetes-associated wounds and/or poorly healing arterial wounds.

In another preferred embodiment, the invention relates to the use of a polypeptide according to SEQ ID No.: 9 for treatment and/or prevention of diseases which are selected from poorly healing diabetes-associated wounds and/or poorly healing arterial wounds.

The term "functional variants" is to be understood as meaning variants of the ACT or AAT polypeptides, respectively, which can be used in accordance with the invention, which variants possess protease inhibitor specificity with regard to capthepsin G or neutrophil elastase which is similar to that of the native ACT polypeptide and AAT polypeptide, respectively. "Functional variants" also comprise ACT or AAT variants which, on a single polypeptide chain, possess both elastase and cathepsin G inhibitory action which is similar to that both of the native ACT polypeptide and AAT polypeptide; such functional variants and nucleic acids encoding these are combinations of ACT and AAT according to the invention.
For example, functional variants of ACT or AAT polypeptides possess at least approximately 70%, in particular at least approx. 80%, especially at least approx. 90%, sequence identity with one of the sequences SEQ ID No. 1, SEQ ID No.: 4, SEQ ID No.: 5, SEQ ID No.: 6, SEQ ID No.: 7 or SEQ ID No.: 9 and have the protease inhibitory activity described above. Functional variants of the polypeptide can also be parts of the polypeptides used in accordance with the invention which, when compared with the native ACT or AAT polypeptides, do exhibit similar protease inhibitor activity, respectively. For example, the first amino acid, i.e. methionine, can be missing in a polypeptide without there being any significant change in the function of the polypeptide. N- and/or C-terminal and/or internal deletions of the polypeptide in the range of approx. 1-60, preferably of approx. 1-30, in particular of approx. 1-15, especially of approx. 1-5, amino acids are also included provided the protease inhibitor specificity remains essentially unaltered as compared with that of the respective native polypeptide. Particular preference is given to deletions which affect the signal peptide, or parts thereof, at the N terminus of an ACT or AAT polypeptide. Examples of such variants, whose protease inhibitor specificity is not significantly altered as compared with that of the native polypeptide, are the polypeptides which are homologous with the polypeptides used in accordance with the invention and which are derived, in particular, from organisms other than humans or mice, preferably from non-human mammals such as monkeys, pigs and rats. Other examples of polypeptides which are encoded by different alleles of the gene, in different individuals or in different organs, and which, when compared with the native polypeptide, do not exhibit any protease inhibitor specificity which is significantly altered as compared with that of the native polypeptide in an organism. Furthermore, a posttranslational or cotranslational modification of the polypeptide chain which is present in the native state can be missing or be altered. In particular, covalently bound sugar residues can be missing or be altered, and activity still being similar. For example, human ACT can be produced in E. coli in an unglycosylated form and the specific enzymatic activity is still retained (e.g. US 5,079,336). Similarly, ACT and AAT and their functional variants may be produced in bacteria or yeast strains to obtain unglycosylated functional variants.

Preferred functional variants are ACT and AAT polypeptides in which the signal peptide, or a part thereof, is deleted, for example a combination of polypeptides according to SEQ ID No. 4 together with SEQ ID No. 7 is especially preferred for use according to the invention. Furthermore, the ACT and AAT which can be used in accordance with the invention can be glycosylated, partially glycosylated or unglycosylated. In a preferred embodiment, the ACT and AAT polypeptides are unglycosylated. Such ACT and AAT polypeptides can be obtained by recombinant expression e.g. in bacteria or in yeast strains. In another preferred embodiment, the ACT and AAT polypeptides are glycosylated. Such ACT and AAT polypeptides can be obtained by isolation from mammalian tissue or by recombinant expression in mammalian cells.

The term "coding nucleic acid" or "nucleic acid encoding..." (an ACT or AAT polypeptide) relates to an RNA or DNA sequence which encodes an ACT or AAT polypeptide which can be used in accordance with the invention or a functional variant thereof or a precursor stage thereof, for example a propolypeptide or a prepropolypeptide. The polypeptide can be encoded by a full-length sequence or any part of the coding sequence as long as the polypeptide is a functional variant.

The term "variants" denotes all the DNA sequences which are complementary to a DNA sequence (reference sequence), which encode polypeptides used in accordance with the invention, especially polypeptides according SEQ ID No. 1, SEQ ID No.: 3, SEQ ID No. 4, SEQ ID No.: 6, SEQ ID No.:7 or SEQ ID No. 9 or their functional variants and which exhibit at least approx. 70%, in particular at least approx. 80%, especially at least approx. 90%, sequence identity with the reference sequence. The term "variants" furthermore denotes all the DNA sequences which are complementary to the reference sequence and which hybridize with the reference sequence under stringent conditions and encode a polypeptide which exhibits essentially the same activity as does the polypeptide encoded by the reference sequence, and also their degenerate forms. It is known that small changes can be present in the sequence of the nucleic acids which can be used in accordance with the invention; for example, without the property of a functional variant being lost, these changes can be brought about by the degeneracy of the genetic code or by nontranslated sequences which are appended at the 5' end and/or the 3' end of the nucleic acid. This invention therefore also encompasses so-called "variants" of the previously described nucleic acids.

The term "stringent hybridization conditions" is to be understood, in particular, as meaning those conditions in which a hybridization takes place, for example, at 60°C in 2.5×SSC buffer, followed by several washing steps at 37°C in a lower buffer concentration, and remains stable.

Sequence identity is understood as degree of identity (% identity) of two sequences, that in the case of polypeptides can be determined by means of for example BlastP 2.0.1 and in the case of nucleic acids by means of for example BLASTN 2.014, wherein the filter is set off and BLOSUM is 62 (Altschul et al., 1997, Nucleic Acids Res., 25:3389-3402). "Sequence homology" is understood as similarity (% positives) of two polypeptide sequences determined by means of for example BlastP 2.0.1 wherein the Filter is set off and BLOSUM is 62 (Altschul et al., 1997, Nucleic Acids Res., 25:3389-3402).

Within the meaning of the present invention, diabetes-associated wounds which heal poorly are to be understood as being skin lesions in mammals and humans suffering from diabetes. Examples of such skin lesions are to be understood as being, in particular, ulcers caused by diabetes, for example ulcus cruris arteriosum, necrobiosis lipoidica and ulcera arteriosa, and delayed wound healing which is caused by arteriosclerotic destruction of the blood vessels.

Within the meaning of the invention, arterial wounds which heal poorly are to be understood, for example, as being ulcera arteriosa and retarded wound healing which is caused by arteriosclerotic destruction of the blood vessels.

Within the meaning of the present invention the function of ACT is to be understood as the activity which ACT exerts onto protease Cathepsin G. The function of ACT also encompasses the activity of ACT in the form of complexes of ACT and Cathepsin G, i.e. the Cathepsin G:ACT complex which binds to receptors, e.g. the serpin- enzyme complex receptor (SECR) (Chen et al., 1993, Neurology 43: 1223-7; Perlmutter et al., 1990 PNAS: 87: 3753-7).

Within the meaning of the present invention, activity of ACT preferably encompasses the binding and inhibition of Cathepsin G protease. Suitable assays to determine the activity of ACT are protease inhibitory assays such as, e.g. Example 2 or Heidtmann et al., 1990, Clin Chem 36: 2077-2081.

Within the meaning of the present invention the function of AAT is to be understood as the binding and inhibition of the protease neutrophil elastase. A suitable assay is described in Rubin et al., 1994, Biochemistry, 33: 7627-7633.

A "similar protease inhibitor activity" within the meaning of the invention is a protease inhibitor activity of a ACT or AAT functional variant that is preferably not less than 0,1%, more preferably not less than 1%, even more preferred not less than 50% and most preferred not less than 90% of the activity of the native enzyme.

The nucleic acids which can be used in accordance with the invention and which encode ACT or AAT polypeptides which can be used in accordance with the invention, or their functional variants, are preferably DNA or RNA, preferably a DNA, in particular a double-stranded DNA. Furthermore, in one embodiment, the sequence of the nucleic acids can be characterized by the fact that it possesses at least one intron and/or a polyA sequence.

In general, preference is given to a double-stranded DNA for expressing the relevant gene, both for preparing a polypeptide which can be used in accordance with the invention and in association with a vector which is applicable in gene therapy and can be used in accordance with the invention, with particular preference being given to the DNA region which encodes the polypeptide. In eukaryotes, this region begins with the first start codon (ATG) which is located in a Kozak sequence (Kozak, 1987, Nucleic. Acids Res. 15:8125-48) and extends to the next stop codon (TAG, TGA or TAA) which is located in the same reading frame as the ATG. In the case of prokaryotes, this region begins with the first AUG (or GUG) after a Shine-Dalgarno sequence and ends with the next stop codon (TAG, TGA or TAA) which is located in the same reading frame as the ATG.

Furthermore, it is possible to use a nucleic acid which has been prepared synthetically for implementing the invention. Thus, the nucleic acid which is used in accordance with the invention can, for example, be synthesized chemically, e.g. in accordance with the phosphotriester method, making use of the DNA sequences described in the sequence listing and/or making use of the protein sequences which are likewise described in this table by referring to the genetic code (see, e.g., Uhlmann, E. & Peyman, A. (1990) Chemical Reviews, 90, 543-584, No. 4).

In a particularly preferred embodiment of the invention, at least one nucleic acid which can be used in accordance with the invention is contained in an expression cassette in a vector, preferably in a vector which is applicable in gene therapy. The invention also comprises the use of a vector expressing a fusion protein useable according to the invention. The vector which is applicable in gene therapy preferably contains tissue-specific, wound-specific or skin-specific, cell cycle-specific, cell type-specific, metabolism-specific or constitutively active regulatory sequences which are functionally linked to the previously described nucleic acid.

Examples of suitable regulable elements which permit constitutive expression in eukaryotes are promoters which are recognized by RNA polymerase III or viral promoters, CMV enhancer, CMV promoter (see also Example 3), SV40 promoter or LTR promoters, e.g. derived from MMTV (mouse mammary tumor virus; Lee et al. (1981) Nature 214, 228-232) and other viral promoter and activator sequences which are derived from, for example, HBV, HCV, HSV, HPV, EBV, HTLV or HIV.

Examples of regulable elements which permit inducible expression in eukaryotes are the tetracycline operator in combination with an appropriate repressor (Gossen M. et al. (1994) Curr. Opin. Biotechnol. 5, 516-20).

The expression of nucleic acids which can be used in accordance with the invention preferably takes place under the control of tissue-specific promoters, with skin-specific promoters, such as the human K10 promoter (Bailleul et al., 1990. Cell 62: 697-708), the human K14 promoter (Vassar et al., 1989, Proc. Natl. Acad. Sci. USA 86: 1563-67) or the bovine cytokeratin IV promoter (Fuchs et al., 1988; The Biology of Wool and Hair (eds.: G.E. Rogers, et al.), pp. 287-309. Chapman and Hall, London/New York) being particularly to be preferred.

Other examples of regulable elements which permit tissue-specific expression in eukaryotes are promoters or activator sequences from promoters or enhancers of those genes which encode proteins which are only expressed in particular cell types.

Examples of regulable elements which permit cell cycle-specific expression in eukaryotes are promoters of the following genes: cdc25A, cyclin A, cyclin E, cdc2, E2F, B-myb and DHFR (Zwicker J. and Müller R. (1997) Trends Genet. 13, 3-6).

Examples of regulable elements which permit metabolism-specific expression in eukaryotes are promoters which are regulated by hypoxia, by glucose deficiency, by phosphate concentration or by heat shock.

An example of a regulable element which permits keratinocyte-specific expression in skin is the FiRE element (Jaakkola et al., 2000, Gen. Ther., 7: 1640-1647). The FiRE element is an AP-1-driven, FGF-inducible response element of the syndecan-1 gene (Jaakkola et al., 1998, FASEB J., 12: 959-9).

In order to enable the nucleic acids which can be used in accordance with the invention to be introduced into a eukaryotic or prokaryotic cell by means of transfection, transformation or infection, and thereby enabling the polypeptide to be expressed, the nucleic acid can be present as a plasmid, or as a part of a viral or non-viral vector. Particularly suitable viral vectors in this context are: baculoviruses, vaccinia viruses, adenoviruses, adeno-associated viruses and herpesviruses. Particularly suitable non-viral vectors in this context are: liposomes, virosomes, cationic lipids and polylysine-conjugated DNA.

Examples of vectors which are applicable in gene therapy are viral vectors, for example adenoviral vectors or retroviral vectors (Lindemann et al., 1997, Mol. Med. 3: 466-76; Springer et al., 1998, Mol. Cell. 2: 549-58). Eukaryotic expression vectors are suitable for use in gene therapy when present in isolated form since naked DNA can penetrate into skin cells when applied topically (Hengge et al., 1996, J. Clin. Invest. 97: 2911-6; Yu et al., 1999, J. Invest. Dermatol. 112: 370-5).

Vectors which are applicable in gene therapy can also be obtained by complexing the nucleic acid which can be used in accordance with the invention with liposomes, since this makes it possible to achieve a very high efficiency of transfection, particularly of skin cells (Alexander and Akhurst, 1995, Hum. Mol. Genet. 4: 2279-85). In lipofection, small, unilamellar vesicles consisting of cationic lipids are prepared by subjecting the liposome suspension to ultrasonication. The DNA is bound ionically on the surface of the liposomes, specifically in a relationship which is such that a positive net charge remains and 100% of the plasmid DNA is complexed by the liposomes. In addition to the DOTMA (1,2-dioleoyloxypropyl-3-trimethylammonium bromide) and DPOE (dioleoylphosphatidylethanolamine) lipid mixtures employed by Felgner et al. (1987, see above), a large number of new lipid formulations have by now been synthesized and tested for their efficiency in the transfection of various cell lines (Behr, J.P. et al. (1989), Proc. Natl. Acad. Sci. USA 86, 6982-6986; Felgner J.H. et al. (1994) J. Biol. Chem. 269, 2550-2561; Gao, X. and Huang L. (1991), Biochim. Biophys. Acta 1189, 195-203). Examples of the new lipid formulations are DOTAP N-[1-(2,3-dioleoyloxy)propyl]-N,N,N-trimethylammonium ethyl sulfate or DOGS (TRANSFECTAM; dioctadecylamidoglycylspermine). The Cytofectin GS 2888 cationic lipids have also proved to be very well suited for transfecting keratinocytes in vitro and in vivo (US 5,777,153; Lewis et al., 1996, Proc. Natl. Acad. Sci. USA, 93: 3176-3181). Auxiliary substances which increase the transfer of nucleic acids into the cell can, for example, be proteins or peptides which are bound to DNA or synthetic peptide-DNA molecules which make it possible to transport the nucleic acid into the nucleus of the cell (Schwartz et al. (1999) Gene Therapy 6, 282; Branden et al. (1999) Nature Biotech. 17, 784). Auxiliary substances also encompass molecules which enable nucleic acids to be released into the cytoplasm of the cell (Planck et al. (1994) J. Biol. Chem. 269, 12918; Kichler et al. (1997) Bioconj. Chem. 8, 213). Liposomes are a pharmaceutically acceptable carrier within the meaning of the present invention. Liposomes comprise multilamellar vesicles (MLVs), small unilamellar vesicles (SUVs) and large unilamellar vesicles (LUVs).

Methods for preparing liposome-nucleic acid complexes are known to the skilled person (e.g. Straubinger et al., 1983, in Methods of Immunology, 101: 512-527; Szoka et al., 1978, Proc. Natl. Acad. Sci. USA, 75: 4194-4198), The term "liposomes" encompasses, for example, liposomal compositions which are disclosed in US 5,422,120, WO 95/13796, WO 94/23697, WO 91/14445 and EP 524,968 B1. Liposomes can be used for nucleic acids usable according to the invention as well as for polypeptides usable according to the invention or for both as a pharmaceutical carrier, preferably they are used as pharmaceutical carriers for the nucleic acids according to the invention. The therapeutically active substance can also be conjugated to the liposome or it can be conjugated to a hydrogel polymer, wherein the hydrogel polymer (or a component of the hydrogel polymer) is conjugated to a liposome or can be enclosed by a liposome. Another especially suitable form of gene therapeutical vectors can be obtained by applying the nucleic acid usable according to the invention to gold particles and apply these topically with the aid of the so called "gene gun" by shooting them into the skin or cells (Example 1; Wang et al., 1999, J. Invest. Dermatol., 112: 775-81, Tuting et al., 1998, J. Invest. Dermatol., 111: 183-8). Nucleic acids encoding AAT and ACT polypeptides may then be applied together at the same time or temporally separated from each other.

The term "liposomes" encompasses, for example, liposomal compositions which are disclosed in US 5,422,120, WO 95/13796, WO 94/23697, WO 91/14445 and EP 524,968 B1. Liposomes can be used as a pharmaceutical carrier for the nucleic acids which can be used in accordance with the invention and the polypeptides which can be used in accordance with the invention; they are preferably used as a pharmaceutical carrier for the nucleic acids which can be used in accordance with the invention. The therapeutically active substance can be conjugated to the liposome or it can be conjugated to a hydrogel polymer, with it being possible for the hydrogel polymer (or a component of the hydrogel polymer) to be conjugated to a liposome or to be enclosed by a liposome. Another particularly preferred form of vector for gene therapy can be obtained by applying the nucleic acid which is used in accordance with the invention to gold particles and using a Gene Gun to administer the particles topically by shooting the loaded particles into the skin or cells (Example 1; Wang et al., 1999, J. Invest. Dermatol., 112: 775-81, Tuting et al., 1998, J. Invest. Dermatol, 111: 183-8). Devices for performing intradermal injection using pressure have been disclosed, for example, in US 5630796.

Another form of vector which is applicable in gene therapy can be prepared by introducing "naked" expression vectors into a biocompatible matrix, for example a collagen matrix. This matrix can, for example, be introduced into diabetes-associated and/or arterial wounds in order to transfect the immigrating cells with the expression vector and to express the polypeptides used in accordance with the invention in the cells (Goldstein and Banadio, US 5,962,427).

For the use of the previously described nucleic acid in gene therapy it is also advantageous if the part of the nucleic acid which encodes the polypeptide contains one or more non-coding sequences, including intron sequences, preferably between the promoter and the start codon for the polypeptide (see Example 1) and/or a polyA sequence, in particular the naturally occurring polyA sequence or an SV40 virus polyA sequence, in particular at the 3' end of the gene since this thereby makes it possible to stabilize the mRNA (Jackson, R.J. (1993) Cell 74, 9-14 and Palmiter, R.D. et al. (1991) Proc. Natl. Acad. Sci.USA 88, 478-482).

For production of the polypeptides, cells can be either prokaryotic or eukaryotic cells. Examples of prokaryotic cells are E. coli, and examples of eukaryotic cells are Saccharomyces cerevisiae or insect cells. Thus, E. coli cells have, for example, proved to be suitable cells for expressing human ACT (Rubin et al., 1990, J. Biol. chem., 265: 1199-1207). The use of E. coli cells for preparing ACT and AAT polypeptides which can be used in accordance with the invention constitutes a preferred embodiment. In a further preferred embodiment the ACT and AAT polypeptides are produced in yeast strains. The polypeptides which can be used in accordance with the invention are prepared, for example, by expressing the previously described nucleic acid in a suitable expression system, as already described above, using methods which are well known to the skilled person. Examples of suitable cells are the E. coli strain DHS, HB101 or BL21, the yeast strain Saccharomyces cerevisiae, the insect cell line Lepidopteran, e.g. from Spodoptera frugiperda, or the animal cells COS, Vero, 293, HaCaT and HeLa, all of which are generally available.

Another preferred embodiment of the invention is the use of an ACT polypeptide or a functional variant thereof in combiantion with an AAT polypeptide or a functional variant thereof which can be used in accordance with the invention in the form of a fusion protein for prevention and/or treatment of diabetes-associated and/or arterial wounds which heal poorly, which fusion protein is prepared using a previously described nucleic acid which can be used in accordance with the invention.

This involves preparing fusion proteins which contain the above-described polypeptides which can be used in accordance with the invention or their functional variants, with the fusion proteins themselves already constituting a functional variant of one of the previously described ACT or AAT polypeptides or only being a functional variant after the fusion moiety has been eliminated. These fusion proteins include, in particular, fusion proteins which have a content of approx. 1-300, preferably approx. 1-200, particularly preferably approx. 1-150, in particular approx. 1-100, and especially approx. 1-50, foreign amino acids. Examples of such peptide sequences are prokaryotic peptide sequences which can be derived, for example, from E. coli galactosidase.

Other preferred examples of peptide sequences for fusion proteins are peptides which facilitate detection of the fusion protein; they include, for example, green fluorescent protein or variants thereof.

The polypeptides which can be used in accordance with the invention can also be prepared synthetically. Thus, the entire polypeptide, or parts thereof, can, for example, be synthesized by means of classical synthesis (Merrifield technique). Particular preference is given to using polypeptides which have been prepared recombinantly using one of the previously described nucleic acids. Furthermore, ACT and AAT polypeptides can be isolated from an organism or from tissue or cells and then used in accordance with the invention. Thus, it is possible, for example, to purify polypeptides which can be used in accordance with the invention from human serum, for example (e.g. Abdullah et al., 1983, Arch. Biochem, Biophys., 225:306-312). Furthermore, it is possible to prepare cell lines from ACT-expressing cells, which cell lines can then be used for isolating ACT. Thus, active ACT can, for example, be prepared by recombinant expression in E. coli cells (Rubin et al., 1990, J. Biol. Chem. 265: 1199-1207).

It is possible to add on at least one "polypeptide tag" for the purpose of purifying the previously described proteins. For example, suitable protein tags enable the proteins which are to be purified to be absorbed with high affinity to a matrix. This is then followed, for example, by the following steps: stringent washing with suitable buffers without eluting the complex to any significant extent, and, subsequently, specific elution of the absorbed complex. Examples of the protein tags which are known to the skilled person are a (His)₆ tag, an Myc tag, a FLAG tag, a hemagglutinin tag, a glutathione transferase (GST) tag, a tag consisting of a an intein flanked by an affinity chitin-binding domain, and a maltose-binding protein (MBP) tag. These protein tags can be located N-terminally, C-terminally and/or internally.

In another embodiment, antibodies or antibody fragments are useable according to the invention wherein one antibody is a catalytic antibody which increases the activity of an ACT polypeptide and a second antibody is a catalytic antibody which increases the activity of an AAT polypeptide . Examples of catalytic antibodies are found for example in Tramontano et al., 1986, Science 234: 1566-70.

The present invention also relates to the use of an alpha 1-antichymotrypsin (ACT) polypeptide, a functional variant thereof and/or a nucleic acid encoding it, or of a cell which is expressing an ACT polypeptide or a nucleic acid encoding it, in combination with an alpha-1-antitrypsin (AAT) polypeptide, a functional variant thereof or a nucleic acid encoding it, or with a cell which is expressing an AAT polypeptide or a nucleic acid encoding it, for producing a pharmaceutical for the treatment and/or prevention of poorly healing diabetes-associated and/or poorly healing arterial wounds.

Preferably, an ACT polypeptide having a sequence selected from the group comprising SEQ ID No.: 1, SEQ ID No.: 6 and SEQ ID No.: 7, or a functional variant thereof or a nucleic acid encoding it, is combined with an AAT polypeptide having a sequence selected from the group comprising SEQ ID No.: 3 and SEQ ID No.: 4, or a functional variant thereof or a nucleic acid encoding it.

In a preferred embodiment, an ACT polypeptide having a sequence according to SEQ ID No.: 7 is combined with an AAT polypeptide according to SEQ ID No.: 4.

In one embodiment, a polypeptide having a sequence according to SEQ ID No.: 9 is used.

In one embodiment, the wounds are a diabetic ulcer or an arterial ulcer, wherein preferably the wounds are a diabetic ulcers.

In one embodiment, the polypeptides are unglycosylated.

In one embodiment, the polypeptides are isolated from tissue.

Preferably, the ACT and AAT polypeptides are administered separately.

In one embodiment, the nucleic acid is employed in the form of an expression vector.

Preferably, the expression vector is a vector applicable in gene therapy.

In a preferred embodiment, the cells are autologous or allogenic cells, wherein, preferably, the cells are skin cells such as keratinocytes, fibroblasts or endothelial cells.

The therapy of the diabetes-associated and/or arterial wounds which heal poorly can be effected in a conventional manner, e.g. using dressings, plasters, compresses or gels which contain the pharmaceuticals which can be used in accordance with the invention. Thus, it is possible to administer the pharmaceuticals topically and locally in order to exert an immediate and direct effect on wound healing. The topical administration of therapeutic compositions can be effected, for example, in the form of a solution, an emulsion, an cream, an ointment, a foam, an aerosol spray, a gel matrix, a sponge, drops or washings. Suitable additives or auxiliary substances are isotonic solutions, such as physiological sodium chloride solutions or sodium alginate, demineralized water, stabilizers, collagen containing substances such as Zyderm II or matrix-forming substances such as povidone. To generate a gel basis, formulations, such as aluminum hydroxide, polyacrylacid derivatives, such as Carbopol®, cellulose derivatives, such as carboxymethyl cellulose are suitable. These gels can be prepared as hydrogels on a water basis or as oleogels with low- and high molecular paraffines or vaseline and/or yellow or white wax. As emulsifier alkali soaps, metal soaps, amine soaps or partial fatty acid esters of sorbitants can be used whereas lipids can be added as vaseline, natural and synthetic waxes, fatty acids, mono-, di-, triglycerides, paraffin, natural oils, such as cocos oil, synthetic fats, such as Miglyol®. These forms of administration are preferred for using at least one ACT polypeptide which can be used in accordance with the invention. The pharmaceuticals according to the invention can also, where appropriate, be administered topically and locally, in the region of the wound, in the form of liposome complexes or gold particle complexes. This form of administration is preferred for vectors which are applicable in gene therapy and which contain a nucleic acid which can be used in accordance with the invention. Topical formulations for ACT and/or AAT polypeptides are well known in the prior art; e.g. in US 6,294,181, US 6,096,327; US 5,008,242, EP 0 512 090, EP 0 432 117 and US 5,190,917.

Furthermore, the treatment can be effected using a transdermal therapeutic system (TTS), which enables the pharmaceuticals according to the invention to be released in a temporally controlled manner. To improve the penetration of the administered drug through the membrane, additives such as ethanol, urea or propylene glycole can be added in addition to polymeric auxiliaries, such as Eudragit®. TTS have been disclosed, for example, in EP 0 944 398 A1, EP 0 916 336 A1, EP 0 889 723 A1 or EP 0 852 493 A1.

In another embodiment, the pharmaceuticals according to the invention can also be administered as a cell expressing a combination of polypeptides of the invention which is then secreted to the wound site. Preferred cells are autologous or allogenic cells, particularly preferred are skin cells such as keratinocytes, fibroblasts or endothelial cells. A suitable carrier for administering those modified cells would be a micro carrier consisting of bio compatible materials, such as, for example a dextran matrix (US 5,980,888).

However, the treatment with the pharmaceuticals according to the invention can also be effected systemically using parenteralia such as injections or infusions or peroralia. Injections can be applied subcutaneously, intradermally, intraepithelially, intrafusally, intramuscularly, intravenous, intracutaneously, intraperitoneally, or intrathecally and can be formulated as solutions, suspensions, as concentrates or lyophilized powder which can be diluted in isotonic solutions. Infusions can also be applied as isotonic solutions or as fat emulsions, as "high tech" formulations such as liposomes, micro emulsions, nanospheres, microspheres, microcapsules. To improve the adsorption of parenteral applied drugs to proteins or polymers and/or to reduce the association of the drug with glass or plastic surfaces, substances such as albumin, organic solvents, plasmaexpander or surface active substances could be used. Suitable auxiliaries for the production of parenteral applied drugs are isotonic substances, such as sodium chloride, isohydric substances, such as sodium hydrogen carbon acid or tensides or surface active substances and emulsifiers, such as Tween®, Cremophor®, or complex inducing substances such as urea and citrate. An alternative parenteral administration would be nasal, oral cavity or rectal administration. For nasal and oral cavity applications, administrations, such as a spray, an ointment, an aerosols or drops which can be inhaled could be used. Suitable propellants would be tetrahydrofluran or heptafluorpropan but manual pressure systems would also be suitable. These propellants could contain surface active substances such as isopropylmyristat. For the rectal administration clyster preparations, such as tablets or capsules or suppositories. As auxiliary substances, Witepsol®, Massa Estarium® or Novata® could be used. A modified form of parenteral release would be the usage of depots which are implanted under the skin which consist preferably on the basis of biological degradable polymers.

A further systemically applied form is the oral administration such as tablets, fizzy tablets, capsules, pellets, dragees, powder, granules, pastilles, chewing gum, drops or suspensions. Suitable auxiliaries are for example starch, lactose, talcum, stearine acid, cellulose, PVP or Aerosil®. Suitable additives are for example flavors, colors, antioxidants, vitamins, sweeteners, such as glucose or aspartam. These oral application systems can also be prepared as retard system by using, for example, Eudragit® or as gastrointestinal therapeutic system or oral osmotic system. The spatial liberation of the drug within the stomach or gut could be varied by the usage of coated tablets with different coats having different solubility.

Preference is given to pharmaceuticals which bring about an increase in the quantity and/or function, particularily the activity, of ACT and AAT polypeptides or functional variants thereof, especially preferably of AAT polypeptides or functional variants thereof. The polypeptides can be prepared synthetically or recombinantly or can be isolated from tissues or cells, with particular preference being given to preparation using an above-described expression system, in particular using E. coli or yeast cells and purification from tissue, particularly serum. The recombinant proteins which have been prepared in this way can also be present as fusion proteins, e.g. so as to facilitate purification or detection.

Another preferred embodiment of a pharmaceutical according to the present invention is the administration of an activating antibody or fragments thereof which increases the activity of an ACT polypeptide in combination with an activating antibody or fragments thereof which increases the activity of an AAT polypeptide , according to the present invention. The application of such a catalytic antibody can be performed as described above.

Administration of the therapeutically acitve compounds ACT and AAT can be performed together or spatiallly and/or temporally separated. It is, for example possible to apply a formulation containing ACT or a functional variant thereof separately from a formulation containing AAT or a functional variant thereof. Preferably ACT and AAT polypeptides are formulated together. The concentrations necessary to exert a therapeutic effect may be identical, similar or different.

In another preferred embodiment, use is made of cells which contain at least one nucleic acid encoding an ACT polypeptide or functional variants thereof or nucleic acids encoding these, in combination with at least one nucleic acid encoding an AAT polypeptide or functional variants thereof or nucleic acids encoding these for producing a pharmaceutical for treatment and/or prevention of diseases which are selected from diabetes-associated wounds which heal poorly and/or arterial wounds which heal poorly.

Particular preference is given to cells which can be used in accordance with the invention and which contain the nucleic acids in the form of an above-described expression vector or vector which is applicable in gene therapy. In another preferred embodiment a cells expressing fusion proteins useable according to the invention, or antibodies or a fragments thereof useable according to the invention, are used. The cells can then be introduced into the wound directly or, where appropriate, combined with suitable carrier systems and/or additives and/or auxiliary substances and then introduced into the wound. Suitable carrier systems have been disclosed, for example, in US 5,980,888, WO 92/06179, EP 0242 270 or WO 90/02796. Preferred cells are autologous or allogenic cells, especially preferred are skin cells, in particular keratinocytes, endothelial cells and fibroblasts. In a preferred embodiment, cells expressing both ACT and AAT polypeptides can be used according to the invention. In another preferred embodiment cells expressing ACT polypeptides or functional variants thereof are combined with cells expressing AAT polypeptides or functional variants thereof.

Particular preference is given to pharmaceuticals, for the gene therapy treatment, which comprise nucleic acids which can be used in accordance with the invention and which are contained in a vector or a cell, as explained above, or which comprise polypeptides which can be used in accordance with the invention.

Preference is given to the treatment and prevention of diabetic ulcer and/or arterial ulcer or of a poorly healing wound in a diabetic patient or of a poorly healing wound in a patient suffering from arteriosclerotic destruction of the blood vessels. Especially preferred are chronic diabetic or arterial wounds. In a even more preferred embodiment, the wounds are diabetic ulcer or an arterial ulcer. In a most preferred embodiment, the wound is a diabetic ulcer.

The invention will now be further clarified with the aid of the following tables and examples without it being restricted thereto.

### Tables and sequences

Table 1: Mean values for the changes in the tensile strength of the wounds in normal and diabetic rats which were treated with mACT (spi2-2) by means of gene therapy, relative to the tensile strength of wounds which were treated with a control vector. The E/C value is the quotient of the absolute tensile strength measured in a rat treated with ACT (E) and the absolute tensile strength measured in a rat treated with a control vector (C).

Table 2: Comparison of the relative amount of active ACT protein within human wound exudates between normally healing wounds and those deriving from different poorly healing wounds as indicated which is an example of a reduced activity selectively occurring in diabetic-associated poorly healing wounds.

SEQ ID No. 1, SEQ ID No.: 3, SEQ ID No. 4, SEQ ID No. 6, SEQ ID No. 7, SEQ ID No. 9 show the sequences of polypeptides which can be used in accordance with the invention.

SEQ ID No. 2, SEQ ID No. 5 and SEQ ID No. 8 show the sequences of nucleic acids which can be used in accordance with the invention.

### Examples

### Example 1: Improving the wound healing in diabetic rats by administering the murine ACT homologue in vivo

Wound healing was investigated after administering the murine ACT gene, according to SEQ ID No. 2, to diabetic male Sprague Dawley rats. In order to quantify the wound healing, the tensile strength of the wounds was investigated, with a higher tensile strength reflecting an improvement in wound healing.

The diabetic rat animal model is an established model system for investigating diabetes-associated wounds which heal poorly (Davidson, Arch. Dermatol. Res. 290: S 1-S 11 ). Since diabetes is accompanied by microangiopathy, this animal model is also suitable for investigating arterially determined disturbances in wound healing.

A suitable expression vector, pMHint, which was prepared on the basis of the vector pMH (S. Hoffman-La Roche), was first of all constructed by inserting intron II of the rat insulin gene into the HindIII cleavage site between the CMV promoter and the multiple cloning site. The mACT cDNA was then cloned into pMHint using the multiple cloning site. For this, the coding region of the mACT cDNA was amplified by PCR (mACT primer 1: 5'GAGGTACCATGGCTTTCATTGCAG 3' and mACT primer 2: 5'GAATCACGTGACCACCTCCTTTGGGGTTGG CTATC 3'), then cut with KpnI and PmlI and ligated to the expression vector pMHint which had been cut with KpnI and PmlI, thereby giving rise to the expression plasmid pMHintACT. pMHint which contained a luciferase gene (pMHIntLuc) was used as the control vector.

In order to induce the diabetes, 4 rats having a bodyweight of 250 - 300 g were injected i.p. with a freshly prepared aqueous solution of streptozotocin (Sigma) (50 mg/kg of bodyweight). The blood sugar of the animals was checked 7 - 9 days after induction, with a blood sugar level value of more than 200 mg/dL confirming the diabetic state.

The 4 diabetic rats and the 4 nondiabetic control animals were subsequently anaesthetized with a mixture consisting of 2% O₂ (2 l/min) and 1.25% isofluran. The back was depilated and 4 sites were marked on the back of each animal for subsequent wounding. In each case 0.5 µg of plasmid DNA immobilized on gold particles (BioRad) was shot into each site at 500 psi using a Helios gene gun (BioRad), with in each case 2 sites being bombarded with the ACT expression vector pMHIntACT and 2 sites being bombarded with the control vector pMHIntLuc and with in each case one bombardment with pMHIntACT being effected anteriorly and one bombardment being effected posteriorly. Incision wounds of 1 cm in length were then made through the bombarded sites and the wounds were closed with wound clips. The wound biopsies were taken after 10 days and the tensile strength of the wounds was determined using an Instron tensiometer in accordance with the manufacturer's instructions and standardized to the cross sectional area of the wounds. Subsequently, the quotient (E/C value) was calculated from the absolute value of the tensile strength of a wound which had been bombarded with pMHInt ACT and the absolute value of the tensile strength of a wound in the same animal which had been bombarded with the control vector pMHIntLuc. The mean of the E/C values was determined and the changes in tensile strength in dependence on mACT thereby ascertained. The means are given in Table 1.

It turned out that the tensile strength of the wounds treated with pMHIntACT was only clearly increased in the diabetic animals whereas the administration of this plasmid had no significant effect in the control animals. This highlights that the deregulation of expression of ACT in poorly healing, diabetes-associated and/or arterial poorly healing wounds can be specifically compensated for by administration of ACT and leads to a pronounced improvement of the wound healing. On the other hand, this treatment was not suitable for improving wound healing in the control animals, in which it was not possible to achieve any significant improvement in wound healing by administering mACT.

### Example 2: Determination of ACT activity in human wound exudates of normally healing wounds, poorly healing venous ulcers, and wounds of diabetic patients

Activity of ACT polypeptides was measured in wound exudates from patients with different poorly healing wounds and compared to the activity found in wound exudates derived from acute normally healing wounds after surgery.

The measurement of activity of ACT polypeptides was performed by an activity assay according to Heidtmann et al., 1990, Clinical Chemistry 36: 2077-2081. The principle of this assay comprises the coating of a 96 well-microtiter plates with cathepsin G. Cathepsin G can bind active ACT molecules from wound exudates. After complexing active ACT polypeptides with cathepsin G, ACT polypeptides are detected by a primary rabbit anti-human antibody against ACT polypeptides, followed by a secondary goat anti-rabbit antibody which is coupled to alkaline phosphatase. The amount of bound, active ACT polypeptide is then detected by the application of a chromogenic substrate for alkaline phosphatase by monitoring the increase in absorbance 490 nm in a Versamax Plate reader (Molecular Devices).

To start this experiment, wound exudates from one patient suffering from a venous ulcer and wound exudates from wounds from 2 diabetic patients were collected for a period of 24 h and 48 h, respectively, as examples for poorly healing wounds. As a control, wound exudates from one acute normally healing patient were also collected for 24 h and 48 h, respectively. Those wounds typically arise in the course of surgeries aiming at a mamma reduction. The wound exudates from poorly healing wounds were collected by vaccum therapy: a vacuum extractor was applied to the corresponding wound together with a sponge which equally distributes the pressure on the wound . The aspirated fluid was collected by a bottle. In contrast, the wound exudates which derive from wounds after the acute surgical control wounds were collected by a drainage system which is located within the subcutaneous tissue of the wound and which usually applied to post-surgical wounds to prevent infections (Redon's suction drainage). Subsequently, collected wound exudates were centrifuged in 10ml Falcon tubes at 1500 rpm at 4°C in a Heraeus Multifuge 3 S-R for 10 min. to remove contaminating cells. The centrifugation was once repeated with the supernatant at 10000 rpm at 4°C for 15 min to complete purification. Purified wound exudates from different patients were than diluted 1:5000, 1:10000, and 1:20000 in PBS-Tween to perform the activity assay. Therefore, 96 well- microtiter plates were first coated with 200 µl bovine serum albumin solution (10 g/l BSA in 50mM NaHCO3, pH 9.6), covered with plate sealers (Qiagen) and incubated for 30 min. at 37 °C. Afterwards, BSA solution was aspirated and microtiter plates were washed 4 times with 250 µl PBS for 2 min. The washing solution was then replaced by 100 µl coupling solution which was prepared by 1000 fold dilution of a stock solution (3.3 g/l cathepsin G (Calbiochem) in 50 mM sodium acetate, pH 5.5 and 0.5 M NaCl). Again the incubation period was 30 min. at 37°C and wells were washed 4 times with 250 µl PBS-Tween for 2 min. To control the coupling of cathepsin G in general, 100 µl of a cathepsin G substrate solution (200 µg/l succinyl-alanyl-alanyl-proplyl-valyl-p-nitroanilide in 0.1 M 4-(2-hydroxyethyl)-1-piperazineethanesulfonate buffer, pH 7.5, 1 M NaCl, 12 % DMSO) were added to the microtiter plate. After an incubation period at 37°C for 100 min., the increase in absorbance at 414 nm was measured in a Versamex plate reader. To control the quantitive amount of cathepsin G coupled to the microtiter plate after the 30-min. incubation period the activity of cathepsin G in the coupling solution was compared with and without incubation in the microtiter plate. Therefore, 100 µl substrate solution (5g/l succinyl-alanyl-alanyl-proplyl-valyl-p-nitroanilide in DMSO) were added to 900 µl of the coupling solution before and after contacting the microtiter plate. The increase in absorbance was messured for both solutions at 414 nm at 25°C for 1 min. Results were within the linerarity of the assay.

Cathepsin G pre- bound microtiter plates were incubated with 100 µl diluted wound exudates from the acute normally healing wounds, wound exudates from the venous ulcer, wound exudates from wounds of the two diabetic patients, and as a control without addition of any wound exudates. Samples from all patients and all concentrations were applied to the microtiter plate in duplicate. The incubation period again took place at 37°C with sealed microtiter plates for 30 min., followed by repeated washing with PBS-Tween. After the incubation period, microtiter plates were incubated with 100 µl rabbit anti- human ACT antibody (Dako; 1:1000 in PBS-Tween, 2.5% dry milk) at 37°C for 30 min. and washed 4 times with PBS-Tween. Incubation of the primary antibody was followed by incubation with a 100 µl of a secondary goat anti- rabbit antibody which was coupled to alkaline phosphatase (Promega, 1:5000 in PBS-Tween, 2.5% dry milk) at 37°C for 30 min., followed by the washing procedure as described above. As a negative control of the antibody, wells on the microtiter plate were prepared which contain either only wound exudates, only primary antibody, only secondary antibody, only wound exudates with primary antibody, only wound exudates with secondary antibody, or only PBS-Tween, respectively. Alkaline phosphatase was detected by incubation at room temperature with 100 µl/well of OPD (Dako; 6.6mg/l containing 420µl/l 30%H₂O₂). The reaction was stopped by adding 100µl of 0,5M H₂SO₄ per well and absorbance was measured at 490nm with the above-described ELSIA reader. In parallel, a calibration series was determined by diluting commercially available ACT (Calbiochem; 1 mg/ml in 20 mM Tris, pH 7.4 and 150 mM NaCl) stepwise. A standard curve was determined by plotting the dilution series (1:5000, 1:8000, 1:10000, 1:12000, 1:20000; 1:30000, 1:40000, 1:50000, 1:80000, 1:100000, and 1:120000) against the absorbance measured at 490 nm. Subsequently, the amount of active ACT polypeptides in the different wound exudates was determined by means of the standard curve. The numerical values were than standardized by setting the mean value of the amount of active ACT polypeptides measured in the wound exudates collected for a period of 24 h from acute normally healing wounds equal to 1. The relative changes of the mean value of active ACT polypeptides measured in the wound exudates from the different patients after different collection periods are depicted in Table 2.

The results clearly show a 3.3- and 2.3-fold reduced amount of active ACT polypeptides in poorly healing wounds of diabetic patients compared to acute normally healing wounds. Moreover, the results demonstrate that the reduction of active ACT is essentially selective to poorly-healing, diabetes-associated wounds as the amount of active ACT in wound exudates of poorly healing venous ulcers is within the increasing range of acute normally healing wounds. This reduction of active ACT in poorly-healing, diabetes associates wounds is attributed to the presence of high amounts of elastase in these wounds.
Together, the experiments 1 and 2 demonstrate that ACT is effective for treating diabetes-associated and arterial badly healing wounds, but is sensitive to inactivation and thus, ACT in combination with AAT surprisingly represents an effective and stable therapeutic for the treatment and/or prevention of diabetes-associated and arterial badly healing wounds.

For prevention and/or treatment, the amount and/or the activity of ACT and AAT, especially of ACT has to be increased in the region of the wound. Indications which are preferably to be treated are diabetic ulcer and arterial ulcer, in particular diabetic ulcer. The administration of a pharmaceutical comprising ACT in combination with AAT is preferably effected topically, preferably by means of gene therapy. The administration can furthermore preferably be effected by means of the topical application of an ACT polypeptide in combination with an AAT polypeptide according to the invention, since the site of action of ACT and AAT polypeptide is extracellular and it is consequently not necessary for the proteins to penetrate into cells. Preferably, the polypeptides are isolated from tissue, particularly serum. In a further preferred embodiment, the polypeptides are produced recombinantly. ACT and AAT polypeptides may be glycosylated or unglycosylated.

It will be apparent to those skilled in the art that various modifications can be made to the compositions and processes of this invention. Thus, it is intended that the present invention cover such modifications and variations, provided they come within the scope of the appended claims and their equivalents.

**Table 1**

| Mean E/C values | |
|---|---|
| Control animals | Diabetic animals |
| 1.142 | 3.397 |

**Table 2**

| **Source of human wound exudate** | **Time Period of wound exudate collection** | **Relative amount of active ACT protein** |
|---|---|---|
| **Surgical wound after mamma reduction** | **24h** | **1** |
| **Surgical wound after mamma reduction** | **48h** | **1.6** |
| **venous ulcer** | **24h** | **1.4** |
| **venous ulcer** | **48h** | **1.2** |
| **chronic wound of diabetic patients** | **24h** | **0.3** |
| **chronic wound of diabetic patient** | **48h** | **0.7** |

## Claims

1. Use of an alpha 1-antichymotrypsin (ACT) polypeptide, a functional variant thereof and/or a nucleic acid encoding it, or of a cell which is expressing an ACT polypeptide or a nucleic acid encoding it, in combination with an alpha-1-antitrypsin (AAT) polypeptide, a functional variant thereof or a nucleic acid encoding it, or with a cell which is expressing an AAT polypeptide or a nucleic acid encoding it, for the treatment and/or prevention of poorly healing diabetes-associated and/or poorly healing arterial wounds.

2. Use according to claim 1 wherein the ACT polypeptide having a sequence selected from the group comprising SEQ ID No.: 1, SEQ ID No.: 6 and SEQ ID No.: 7, or a functional variant thereof or a nucleic acid encoding it, is combined with an AAT polypeptide having a sequence selected from the group comprising SEQ ID No.: 3 and SEQ ID No.: 4, or a functional variant thereof or a nucleic acid encoding it.

3. Use according to claim 1 or claim 2 wherein an ACT polypeptide having a sequence according to SEQ ID No.: 7 is combined with an AAT polypeptide according to SEQ ID No.: 4.

4. Use according to claim 1 wherein a polypeptide having a sequence according to SEQ ID No.: 9 is used.

5. Use according to one of claims 1 to 4, wherein the wounds are a diabetic ulcer or an arterial ulcer.

6. Use according to claim 5, wherein the wounds are a diabetic ulcers.

7. Use according to at least one of claims 1 to 4, wherein the polypeptides are unglycosylated.

8. Use according to at least one of claims 1 to 4, wherein the polypeptides are isolated from tissue.

9. Use according to at least one of claims 1 to 3, wherein the ACT and AAT polypeptides are administered separately.

10. Use according to claims 1, wherein the nucleic acid is employed in the form of an expression vector.

11. Use according to claims 10, wherein the expression vector is a vector applicable in gene therapy.

12. Use according to claim 1, wherein the cells are autologous or allogenic cells.

13. Use according to claim 12, wherein the cells are skin cells such as keratinocytes, fibroblasts or endothelial cells.

14. Method of manufacturing a pharmaceutical composition for treatment, and/or prevention of diseases selected from poorly healing diabetes-associated wounds and/or poorly healing arterial wounds wherein an ACT polypeptide, or a nucleic acid coding for the ACT polypeptide, or a cell expressing the ACT polypeptide or a nucleic acid coding for the ACT polypeptide, is combined with an AAT polypeptide, or a nucleic acid coding for the AAT polypeptide, or a cell expressing the AAT polypeptide or a nucleic acid coding for the AAT polypeptide.


SEQUENCE LISTING

<110>  SWITCH Biotech Aktiengesellschaft

<120>  Use of alpha-1-antichymotrypsin polypeptides, or nucleic acids enco-
ding them, in combination with alpha-1-antitrypsin polypeptides, or nucleic
acids encoding them, for treatment and/or prevention of diabetes-associated
and/or arterial poorly healing wounds.

<130>  FB11957

<160>  9

<170>  PatentIn version 3.1

<210>  1
<211>  418
<212>  PRT
<213>  Mus musculus

<400>  1

Met Ala Phe Ile Ala Ala Leu Gly Leu Leu Met Ala Arg Ile Cys Pro
1               5                   10                  15

Ala Val Leu Ser Phe Pro Asp Gly Thr Leu Gly Met Asp Ala Ala Val
            20                  25                  30

Gln Glu Asp His Asp Asn Gly Thr Gln Leu Asp Ser Leu Thr Leu Ala
        35                  40                  45

Ser Ile Asn Thr Asp Phe Ala Phe Ser Leu Tyr Lys Glu Leu Val Leu
    50                  55                  60

Lys Asn Pro Asp Thr Asn Ile Val Phe Ser Pro Leu Ser Ile Ser Ala
65                  70                  75                  80

Ala Leu Ala Leu Val Ser Leu Gly Ala Lys Gly Asn Thr Leu Glu Glu
                85                  90                  95

Ile Leu Glu Gly Leu Lys Phe Asn Leu Thr Glu Thr Ser Glu Ala Asp
            100                 105                 110

Ile His Gln Gly Phe Gly His Leu Leu Gln Arg Leu Asn Gln Pro Lys
        115                 120                 125

Asp Gln Val Gln Ile Ser Thr Gly Ser Ala Leu Phe Ile Glu Lys Arg
    130                 135                 140

Gln Gln Ile Leu Thr Glu Phe Gln Glu Lys Ala Lys Thr Leu Tyr Gln
145                 150                 155                 160

Ala Glu Ala Phe Thr Ala Asp Phe Gln Gln Pro Arg Gln Ala Lys Lys

                165                 170                 175

Leu Ile Asn Asp Tyr Val Arg Lys Gln Thr Gln Gly Met Ile Lys Glu
            180                 185                 190

Leu Val Ser Asp Leu Asp Lys Arg Thr Leu Met Val Leu Val Asn Tyr
        195                 200                 205

Ile Tyr Phe Lys Ala Lys Trp Lys Val Pro Phe Asp Pro Leu Asp Thr
    210                 215                 220

Phe Lys Ser Glu Phe Tyr Ala Gly Lys Arg Arg Pro Val Ile Val Pro
225                 230                 235                 240

Met Met Ser Met Glu Asp Leu Thr Thr Pro Tyr Phe Arg Asp Glu Glu
                245                 250                 255

Leu Ser Cys Thr Val Val Glu Leu Lys Tyr Thr Gly Asn Ala Ser Ala
            260                 265                 270

Leu Phe Ile Leu Pro Asp Gln Gly Arg Met Gln Gln Val Glu Ala Ser
        275                 280                 285

Leu Gln Pro Glu Thr Leu Arg Lys Trp Lys Asn Ser Leu Lys Pro Arg
    290                 295                 300

Met Ile Asp Glu Leu His Leu Pro Lys Phe Ser Ile Ser Thr Asp Tyr
305                 310                 315                 320

Ser Leu Glu Asp Val Leu Ser Lys Leu Gly Ile Arg Glu Val Phe Ser
                325                 330                 335

Thr Gln Ala Asp Leu Ser Ala Ile Thr Gly Thr Lys Asp Leu Arg Val
            340                 345                 350

Ser Gln Val Val His Lys Ala Val Leu Asp Val Ala Glu Thr Gly Thr
        355                 360                 365

Glu Ala Ala Ala Ala Thr Gly Val Lys Phe Val Pro Met Ser Ala Lys
    370                 375                 380

Leu Tyr Pro Leu Thr Val Tyr Phe Asn Arg Pro Phe Leu Ile Met Ile
385                 390                 395                 400

Phe Asp Thr Glu Thr Glu Ile Ala Pro Phe Ile Ala Lys Ile Ala Asn
                405                 410                 415

Pro Lys

<210> 2
<211> 2035
<212> DNA
<213> Mus musculus

<400> 2
cacgagcaca gaggctggca gctggctggt ttcagctctg cagactgcag aacacagaag 60

atggctttca ttgcagctct ggggctcttg atggctagga tctgccctgc tgtcctctcc 120

ttcccagatg gcacgttggg aatggatgct gcagtccaag aagaccatga caatgggaca 180

caactggaca gtctcacatt ggcctccatc aacactgact ttgccttcag cctctacaag 240

gagctggttt tgaagaatcc agatacaaat attgtcttct ccccacttag catctcagcg 300

gccttggccc tcgtgtccct gggagcaaag ggcaacaccc tggaagagat tctagaaggt 360

ctcaagttca atcttacaga gacctctgag gcagacatcc accagggctt tgggcacctc 420

ctacagaggc tcaaccagcc aaaggaccag gtacagatca gcacgggtag tgccctgttt 480

attgaaaagc gccagcagat cctgacagaa ttccaggaga aggcaaagac tctgtaccag 540

gctgaggcct tcacagcaga cttccagcag cctcgacagg ccaaaaagct catcaatgac 600

tatgtgagga aacagaccca ggggatgatc aaggaactgg tctcagacct ggataaaagg 660

acattgatgg tgctggtgaa ttatatctac tttaaagcca aatggaaggt gccctttgac 720

cctcttgaca cgttcaagtc tgagttctac gcgggcaaga ggaggcccgt gatagtgccc 780

atgatgagca tggaggacct gaccacaccc tacttccgag atgaggagct gtcctgcact 840

gtggtggagc tgaagtacac aggaaatgcc agcgccctgt tcatcctccc tgaccagggc 900

aggatgcagc aggtggaagc cagcttacaa ccagagaccc tgaggaagtg gaagaattct 960

ctgaaaccca ggatgataga tgagctccac ctgcccaagt tctccatctc caccgactac 1020

agcctggagg atgtcctttc aaagctgggc atcagggaag tcttctccac acaggctgac 1080

ctgtctgcaa tcacaggaac caaggatctg agagtctctc aggtggtcca caaggctgtg 1140

ctggatgtgg ctgagacagg cacagaagca gctgctgcca ctggagtcaa atttgtccca 1200

atgtctgcga aactgtaccc tctgactgta tatttcaatc ggcctttcct gataatgatc 1260

tttgacacag aaactgaaat tgcccccttt atagccaaga tagccaaccc caaatgagac 1320

tagaactccc caagtgttga cgcttcttcc cgggagccag gcattgagcc tgtctgtggg 1380

tctccatgtg cattttggct tccatgctct gcttggcctt ggcatgcctg gattagatag 1440

tgactaactg tgttataacc tcatgtacag acatccctgt gggaagtcag tgccgtgctc 1500

ccagacttct tggtagcact agcccatgtt cctgagcctg aaatttgtct tgtcccctac 1560

ccctgctctc tccctgtatc tgcctccacc caaaagcctg ggccccatca agtaagctca 1620

gtcccagttt agactctggt tatgtctccc ttcagccttg ctgtcttgat gggactgtgc 1680

aaccttacag gccaacccat atggaccaag aggaaagctt ggctggcccg tgtctatccg 1740

cagcatctag gactacttgg tgcccagtct gcctcactct ttcatttctc caggctttgc 1800

ctccaggcac tgcccccctg caggatctcc tcactttgtc cacatctggc acagagttag 1860

agccctcact tctgcagctg catggggtct gtgggtcaga gcagatcctt tcccctggca 1920

ctcctactta gaacaaagta gcctttcttt tagttcccag ctgaccaacc tcacacaaaa 1980

gaggaacacc aaccagaata aagaggtagg agcaaaggat caataaacat gtaac 2035

<210> 3
<211> 418
<212> PRT
<213> Homo sapiens

<400> 3

<210> 4
<211> 398
<212> PRT
<213> Homo sapiens

<400> 4

Val Ser Leu Ala Glu Asp Pro Gln Gly Asp Ala Ala Gln Lys Thr Asp
1               5                   10                  15

Thr Ser His His Asp Gln Asp His Pro Thr Phe Asn Lys Ile Thr Pro
            20                  25                  30

Asn Leu Ala Glu Phe Ala Phe Ser Leu Tyr Arg Gln Leu Ala His Gln
        35                  40                  45

Ser Asn Ser Thr Asn Ile Phe Phe Ser Pro Val Ser Ile Ala Thr Ala
    50                  55                  60

Phe Ala Met Leu Ser Leu Gly Thr Lys Ala Asp Thr His Asp Glu Ile
65                  70                  75                  80

Leu Glu Gly Leu Asn Phe Asn Leu Thr Glu Ile Pro Glu Ala Gln Ile
                85                  90                  95

His Glu Gly Phe Gln Glu Leu Leu Arg Thr Leu Asn Gln Pro Asp Ser
            100                 105                 110

Gln Leu Gln Leu Thr Thr Gly Asn Gly Leu Phe Leu Ser Glu Gly Leu
        115                 120                 125

Lys Leu Val Asp Lys Phe Leu Glu Asp Val Lys Lys Leu Tyr His Ser
    130                 135                 140

Glu Ala Phe Thr Val Asn Phe Gly Asp Thr Glu Glu Ala Lys Lys Gln
145                 150                 155                 160

Ile Asn Asp Tyr Val Glu Lys Gly Thr Gln Gly Lys Ile Val Asp Leu
                165                 170                 175

Val Lys Glu Leu Asp Arg Asp Thr Val Phe Ala Leu Val Asn Tyr Ile
            180                 185                 190

Phe Phe Lys Gly Lys Trp Glu Arg Pro Phe Glu Val Lys Asp Thr Glu
        195                 200                 205

Glu Glu Asp Phe His Val Asp Gln Val Thr Thr Val Lys Val Pro Met
    210                 215                 220

Met Lys Arg Leu Gly Met Phe Asn Ile Gln His Cys Lys Lys Leu Ser
225                 230                 235                 240

Ser Trp Val Leu Leu Met Lys Tyr Leu Gly Asn Ala Thr Ala Ile Phe
                245                 250                 255

Phe Leu Pro Asp Glu Gly Lys Leu Gln His Leu Glu Asn Glu Leu Thr
            260                 265                 270

His Asp Ile Ile Thr Lys Phe Leu Glu Asn Glu Asp Arg Arg Ser Ala
        275                 280                 285

Ser Leu His Leu Pro Lys Leu Ser Ile Thr Gly Thr Tyr Asp Leu Lys
    290                 295                 300

Ser Val Leu Gly Gln Leu Gly Ile Thr Lys Val Phe Ser Asn Gly Ala
305                 310                 315                 320

Asp Leu Ser Gly Val Thr Glu Glu Ala Pro Leu Lys Leu Ser Lys Ala
                325                 330                 335

Val His Lys Ala Val Leu Thr Ile Asp Glu Lys Gly Thr Glu Ala Ala
            340                 345                 350

Gly Ala Met Phe Leu Glu Ala Ile Pro Met Ser Ile Pro Pro Glu Val
        355                 360                 365

Lys Phe Asn Lys Pro Phe Val Phe Leu Met Ile Glu Gln Asn Thr Lys
    370                 375                 380

Ser Pro Leu Phe Met Gly Lys Val Val Asn Pro Thr Gln Lys
385                 390                 395

<210>  5
<211>  1352
<212>  DNA
<213>  Homo sapiens

<400>  5
ctgggacagt gaatcgacaa tgccgtcttc tgtctcgtgg ggcatcctcc tgctggcagg      60

cctgtgctgc ctggtccctg tctccctggc tgaggatccc cagggagatg ctgcccagaa     120

gacagataca tcccaccatg atcaggatca cccaaccttc aacaagatca cccccaacct     180

ggctgagttc gccttcagcc tataccgcca gctggcacac cagtccaaca gcaccaatat     240

cttcttctcc ccagtgagca tcgctacagc ctttgcaatg ctctccctgg ggaccaaggc 300

tgacactcac gatgaaatcc tggagggcct gaatttcaac ctcacggaga ttccggaggc 360

tcagatccat gaaggcttcc aggaactcct ccgtaccctc aaccagccag acagccagct 420

ccagctgacc accggcaatg gcctgttcct cagcgagggc ctgaagctag tggataagtt 480

tttggaggat gttaaaaagt tgtaccactc agaagccttc actgtcaact tcggggacac 540

cgaagaggcc aagaaacaga tcaacgatta cgtggagaag ggtactcaag ggaaaattgt 600

ggatttggtc aaggagcttg acagagacac agtttttgct ctggtgaatt acatcttctt 660

taaaggcaaa tgggagagac cctttgaagt caaggacacc gaggaagagg acttccacgt 720

ggaccaggtg accaccgtga aggtgcctat gatgaagcgt ttaggcatgt ttaacatcca 780

gcactgtaag aagctgtcca gctgggtgct gctgatgaaa tacctgggca atgccaccgc 840

catcttcttc ctgcctgatg aggggaaact acagcacctg gaaaatgaac tcacccacga 900

tatcatcacc aagttcctgg aaaatgaaga cagaaggtct gccagcttac atttacccaa 960

actgtccatt actggaacct atgatctgaa gagcgtcctg ggtcaactgg gcatcactaa 1020

ggtcttcagc aatggggctg acctctccgg ggtcacagag gaggcacccc tgaagctctc 1080

caaggccgtg cataaggctg tgctgaccat cgacgagaaa gggactgaag ctgctggggc 1140

catgttttta gaggccatac ccatgtctat cccccccgag gtcaagttca acaaaccctt 1200

tgtcttctta atgattgaac aaaataccaa gtctcccctc ttcatgggaa aagtggtgaa 1260

tcccacccaa aaataactgc ctctcgctcc tcaacccctc ccctccatcc ctggccccct 1320

ccctggatga cattaaagaa gggttgagct gg 1352

<210> 6
<211> 423
<212> PRT
<213> Homo sapiens

<400> 6

<210> 7
<211> 400
<212> PRT
<213> Homo sapiens

<400> 7

His Pro Asn Ser Pro Leu Asp Glu Glu Asn Leu Thr Gln Glu Asn Gln
1               5                   10                  15

Asp Arg Gly Thr His Val Asp Leu Gly Leu Ala Ser Ala Asn Val Asp
            20                  25                  30

Phe Ala Phe Ser Leu Tyr Lys Gln Leu Val Leu Lys Ala Pro Asp Lys
        35                  40                  45

Asn Val Ile Phe Ser Pro Leu Ser Ile Ser Thr Ala Leu Ala Phe Leu
    50                  55                  60

Ser Leu Gly Ala His Asn Thr Thr Leu Thr Glu Ile Leu Lys Gly Leu
65                  70                  75                  80

Lys Phe Asn Leu Thr Glu Thr Ser Glu Ala Glu Ile His Gln Ser Phe
                85                  90                  95

Gln His Leu Leu Arg Thr Leu Asn Gln Ser Ser Asp Glu Leu Gln Leu
            100                 105                 110

Ser Met Gly Asn Ala Met Phe Val Lys Glu Gln Leu Ser Leu Leu Asp
        115                 120                 125

Arg Phe Thr Glu Asp Ala Lys Arg Leu Tyr Gly Ser Glu Ala Phe Ala
    130                 135                 140

Thr Asp Phe Gln Asp Ser Ala Ala Ala Lys Lys Leu Ile Asn Asp Tyr
145                 150                 155                 160

Val Lys Asn Gly Thr Arg Gly Lys Ile Thr Asp Leu Ile Lys Asp Leu
                165                 170                 175

Asp Ser Gln Thr Met Met Val Leu Val Asn Tyr Ile Phe Phe Lys Ala
            180                 185                 190

Lys Trp Glu Met Pro Phe Asp Pro Gln Asp Thr His Gln Ser Arg Phe
        195                 200                 205

Tyr Leu Ser Lys Lys Lys Trp Val Met Val Pro Met Met Ser Leu His
    210                 215                 220

His Leu Thr Ile Pro Tyr Phe Arg Asp Glu Glu Leu Ser Cys Thr Val
225                 230                 235                 240

Val Glu Leu Lys Tyr Thr Gly Asn Ala Ser Ala Leu Phe Ile Leu Pro
                245                 250                 255

Asp Gln Asp Lys Met Glu Glu Val Glu Ala Met Leu Leu Pro Glu Thr
            260                 265                 270

Leu Lys Arg Trp Arg Asp Ser Leu Glu Phe Arg Glu Ile Gly Glu Leu
        275                 280                 285

Tyr Leu Pro Lys Phe Ser Ile Ser Arg Asp Tyr Asn Leu Asn Asp Ile
    290                 295                 300

Leu Leu Gln Leu Gly Ile Glu Glu Ala Phe Thr Ser Lys Ala Asp Leu
305                 310                 315                 320

Ser Gly Ile Thr Gly Ala Arg Asn Leu Ala Val Ser Gln Val Val His
                325                 330                 335

Lys Ala Val Leu Asp Val Phe Glu Glu Gly Thr Glu Ala Ser Ala Ala
            340                 345                 350

Thr Ala Val Lys Ile Thr Leu Leu Ser Ala Leu Val Glu Thr Arg Thr
        355                 360                 365

Ile Val Arg Phe Asn Arg Pro Phe Leu Met Ile Ile Val Pro Thr Asp
    370                 375                 380

Thr Gln Asn Ile Phe Phe Met Ser Lys Val Thr Asn Pro Lys Gln Ala
385                 390                 395                 400

<210>  8
<211>  1522
<212>  DNA
<213>  Homo sapiens

<400>  8
cagagttgag aatggagaga atgttacctc tcctgactct ggggctcttg gcggctgggt      60

tctgccctgc tgtcctctgc caccctaaca gcccacttga cgaggagaat ctgacccagg     120

agaaccaaga ccgagggaca cacgtggacc tcggattagc ctccgccaac gtggacttcg     180

ctttcagcct gtacaagcag ttagtcctga aggcccctga taagaatgtc atcttctccc     240

cactgagcat ctccaccgcc ttggccttcc tgtctctggg ggcccataat accaccctga     300

cagagattct caaaggcctc aagttcaacc tcacggagac ttctgaggca gaaattcacc     360

agagcttcca gcacctcctg cgcaccctca atcagtccag cgatgagctg cagctgagta     420

tgggaaatgc catgtttgtc aaagagcaac tcagtctgct ggacaggttc acggaggatg     480

ccaagaggct gtatggctcc gaggcctttg ccactgactt tcaggactca gctgcagcta     540

agaagctcat caacgactac gtgaagaatg gaactagggg gaaaatcaca gatctgatca     600

aggaccttga ctcgcagaca atgatggtcc tggtgaatta catcttcttt aaagccaaat     660

gggagatgcc ctttgacccc caagatactc atcagtcaag gttctacttg agcaagaaaa     720

agtgggtaat ggtgcccatg atgagtttgc atcacctgac tataccttac ttccgggacg     780

aggagctgtc ctgcaccgtg gtggagctga agtacacagg caatgccagc gcactcttca     840

tcctccctga tcaagacaag atggaggaag tggaagccat gctgctccca gagaccctga     900

agcggtggag agactctctg gagttcagag agataggtga gctctacctg ccaaagtttt     960

ccatctcgag ggactataac ctgaacgaca tacttctcca gctgggcatt gaggaagcct    1020

tcaccagcaa ggctgacctg tcagggatca caggggccag gaacctagca gtctcccagg    1080

tggtccataa ggctgtgctt gatgtatttg aggagggcac agaagcatct gctgccacag    1140

cagtcaaaat caccctcctt tctgcattag tggagacaag gaccattgtg cgtttcaaca    1200

ggcccttcct gatgatcatt gtccctacag acacccagaa catcttcttc atgagcaaag    1260

tcaccaatcc caagcaagcc tagagcttgc catcaagcag tggggctctc agtaaggaac    1320

ttggaatgca agctggatgc ctgggtctct gggcacagcc tggcccctgt gcaccgagtg    1380

gccatggcat gtgtggccct gtctgcttat ccttggaagg tgacagcgat tccctgtgaa    1440

gctctcacac gcacaggggc ccatggactc ttcagtctgg agggtcctgg cctcctgaca    1500

gcaataaata atttcgttgg cc                                              1522

<210>  9
<211>  400
<212>  PRT
<213>  Artificial Sequence

<220>
<223>  Lex032 polypeptide; ACT/AAT hybrid

<400>  9

His Pro Asn Ser Pro Leu Asp Glu Glu Asn Leu Thr Gln Glu Asn Gln
1               5                   10                  15

Asp Arg Gly Thr His Val Asp Leu Gly Leu Ala Ser Ala Asn Val Asp
            20                  25                  30

Phe Ala Phe Ser Leu Tyr Lys Gln Leu Val Leu Lys Ala Pro Asp Lys
        35                  40                  45

Asn Val Ile Phe Ser Pro Leu Ser Ile Ser Thr Ala Leu Ala Phe Leu
    50                  55                  60

Ser Leu Gly Ala His Asn Thr Thr Leu Thr Glu Ile Leu Lys Gly Leu
65                  70                  75                  80

Lys Phe Asn Leu Thr Glu Thr Ser Glu Ala Glu Ile His Gln Ser Phe
                85                  90                  95

Gln His Leu Leu Arg Thr Leu Asn Gln Ser Ser Asp Glu Leu Gln Leu
            100                 105                 110

Ser Met Gly Asn Ala Met Phe Val Lys Glu Gln Leu Ser Leu Leu Asp
        115                 120                 125

Arg Phe Thr Glu Asp Ala Lys Arg Leu Tyr Gly Ser Glu Ala Phe Ala
    130                 135                 140

Thr Asp Phe Gln Asp Ser Ala Ala Ala Lys Lys Leu Ile Asn Asp Tyr
145                 150                 155                 160

Val Lys Asn Gly Thr Arg Gly Lys Ile Thr Asp Leu Ile Lys Asp Leu
                165                 170                 175

Asp Ser Gln Thr Met Met Val Leu Val Asn Tyr Ile Phe Phe Lys Ala
            180                 185                 190

Lys Trp Glu Met Pro Phe Asp Pro Gln Asp Thr His Gln Ser Arg Phe
        195                 200                 205

Tyr Leu Ser Lys Lys Lys Trp Val Met Val Pro Met Met Ser Leu His
    210                 215                 220

His Leu Thr Ile Pro Tyr Phe Arg Asp Glu Glu Leu Ser Cys Thr Val
225                 230                 235                 240

Val Glu Leu Lys Tyr Thr Gly Asn Ala Ser Ala Leu Phe Ile Leu Pro
                245                 250                 255

Asp Gln Asp Lys Met Glu Glu Val Glu Ala Met Leu Leu Pro Glu Thr
            260                 265                 270

Leu Lys Arg Trp Arg Asp Ser Leu Glu Phe Arg Glu Ile Gly Glu Leu
        275                 280                 285

Tyr Leu Pro Lys Phe Ser Ile Ser Arg Asp Tyr Asn Leu Asn Asp Ile
    290                 295                 300

Leu Leu Gln Leu Gly Ile Glu Glu Ala Phe Thr Ser Lys Ala Asp Leu
305                 310                 315                 320

Ser Gly Ile Thr Gly Ala Arg Asn Leu Ala Val Ser Gln Val Val His
                325                 330                 335

Lys Ala Val Leu Asp Val Phe Glu Glu Gly Thr Glu Ala Ser Ala Ala
            340                 345                 350

Thr Ala Val Lys Ile Ile Pro Met Ser Ile Pro Val Glu Thr Arg Thr
        355                 360                 365

Ile Val Arg Phe Asn Arg Pro Phe Leu Met Ile Ile Val Pro Thr Asp
    370                 375                 380

Thr Gln Asn Ile Phe Phe Met Ser Lys Val Thr Asn Pro Lys Gln Ala
385                 390                 395                 400